# EUROPEAN PATENT APPLICATION

(11) **EP 4 800 385 A1**
(43) Date of publication of application: **02.09.2026**
(21) Application number: 26161481.2
(22) Date of filing: 27.02.2026
(51) Int. Cl.: G01N 27/48, G01N 27/403

(54) **METHOD FOR DETERMINING AMPHETAMINE IN THE PRESENCE OF CAFFEINE AND PARACETAMOL**

(30) Priority: 27.02.2025 PL 45134125
(71) Applicant: Uniwersytet Lodzki, 90-136 Lodz (PL)
(72) Inventor: Póltorak, Lukasz, 90-136 Lódz (PL); Rudnicki, Konrad, 90-136 Lódz (PL); Kwaczynski, Karolina, 90-236 Lódz (PL); Borgul, Karolina, 90-136 Lódz (PL); Mikolajczyk, Aleksandra, 90-136 Lódz (PL); Skrzypek, Slawomira, 94-122 Lódz (PL); Marciniak, Karolina, 95-100 Zgierz (PL)
(74) Representative: Kicinska-Fujawa, Alicja

(57) **Abstract**

The present invention relates to a rapid, simple, quantitative and qualitative method for the determination of amphetamine.

The method is characterized in that two pairs of electrodes are introduced into an appropriately designed electrochemical cell comprising two parts, respectively filled with an organic phase being a solution of a supporting electrolyte and an aqueous phase containing a solution of a supporting electrolyte that dissociates into ions with a low partition coefficient. One pair of reference electrodes in the form of a wire coated with Ag/AgCl and one pair of auxiliary electrodes made of platinum. All the electrodes are then connected to a potentiostat, which enables potentiostatic polarisation of the liquid-liquid interface. The amphetamine sample under investigation is dissolved in the aqueous phase containing a solution of a supporting electrolyte with a pH below the pKa of amphetamine, and its controlled interfacial transfer across the liquid-liquid interface occurs. The transfer of an amphetamine molecule is recorded using electroanalytical methods such as voltammetry. The value of the potential applied from an external polarisation source provides qualitative information, whilst the Faraday currents recorded provide quantitative information. The determination of the concentration of the amphetamine being tested is based on standard calibration methods.

## Description

The invention relates to a method for the rapid, straightforward, quantitative and qualitative determination of amphetamine. The method is based on the electrochemical transfer of charged amphetamine molecules dissolved in aqueous solutions between two immiscible electrolyte solutions, the liquid-liquid interface of which becomes polarised. This invention could potentially be used in forensic laboratories, in field analyses carried out by law enforcement agencies, and in the context of parental supervision. The essential components are two immiscible solutions, a measuring vessel, a set of electrodes, and a potentiostat or galvanostat.

In an article entitled 'Evaluation of the lipophilic properties of opioids, amphetamine-like drugs, and metabolites through electrochemical studies at the interface between two immiscible solutions', Rubin Gulaboski et al. (Rubin Gulaboski, M. Natalia D.S. Cordeiro, Nuno Milhazes, Jorge Garrido, Fernanda Borges, Miguel Jorge, Carlos M. Pereira, Ivan Bogeski, Aluska Heiguera Morales, Blaze Naumoski, A. Fernando Silva, Anal. Biochem. 361 (2007) 236-243) reported that amphetamine exhibits activity at polarised liquid interfaces. They also determined the partition coefficients of the ionised forms of various opioids, amphetamine-like drugs (including amphetamine itself) and their metabolites, by conducting studies at the liquid-liquid interface. This invention is based on polarised liquid-liquid phase boundaries and enables the analytical determination of amphetamine in real samples of illicitly traded substances, even in the presence of interfering substances such as caffeine and paracetamol. This method provides rapid, simple and direct detection of amphetamine, with a lower limit of detection of approximately 1 µM.

In the scientific paper entitled 'Electrochemical detection of 3,4-methylenedioxymethamphetamine (ecstasy) using a boron-doped diamond electrode with differential pulse voltammetry: Simple and fast screening method for application in forensic analysis' by (K. R. Teófilo, L. C. Arantes, P. A. Marinho, A. A. Macedo, D. M. Pimentel, D. P. Rocha, A. C. de Oliveira, E. M. Richter, R. A. A. Munoz, W. T. P. dos Santos, Microchemical Journal, 157, 2020, 105088*) the authors describe* an electrochemical sensor based on a conventional solid electrode - a boron-doped diamond electrode - which was used for the determination of amphetamine. The principle behind such a sensor is to detect the electrical currents produced by the oxidation of amphetamine (the oxidation of the primary amine that forms part of the amphetamine molecule's structure). Analysis of the recorded signals enables the qualitative and quantitative determination of this substance. The use of conventional solid electrodes other than boron-doped diamond electrodes is very limited due to the very high potential at which amphetamine undergoes oxidation (approx. 1.7 V), which lies beyond the range of other carbon electrodes.

In another publication entitled 'Derivatisation of amphetamine to enable its electrochemical detection in illicit drug seizures' (M. Parrilla, N. F. Montiel, F. Van Durme, K. De Wael, Sensors and Actuators B: Chemical, 337, 2021, 129819*) the authors describe* a process for the derivatisation of amphetamine for its determination on screen-printed electrodes. This method is based on the analysis of currents and potentials associated with the transfer of the amphetamine cation from the aqueous phase to the organic phase without carrying out additional processes that interfere with the structure of the analyte molecule. Unlike the detection of amine compounds on conventional solid-state electrodes, the use of a liquid-liquid phase boundary relies on the interphase transfer of amphetamine ions rather than on oxidation or reduction reactions. Thanks to this approach, a system based on immiscible liquids enables the selective detection of amphetamine, eliminating interference from substances such as paracetamol and caffeine, which can undergo redox reactions at solid electrodes.

A number of existing solutions in this field have been disclosed in numerous patent documents.

Patent specification No. US5328828A describes an immunoassay capable of detecting the presence of amphetamine in a sample suspected of containing amphetamine and/or methamphetamine by using at least two conjugates, each comprising a functionally similar tag bound respectively to an amphetamine analogue and a methamphetamine analogue, and antibodies against amphetamine and methamphetamine, wherein at least one of the antibodies is a monoclonal antibody.

In turn, US2013177994A1 describes methods for the chiral separation and quantitative determination of the d- and l-enantiomers of amphetamine and methamphetamine in body fluids and tissues, using extraction and elution on a chromatographic column.

Patent KR100902571B1 relates to the simultaneous determination of dimethylamphetamine, amphetamine and their metabolites using liquid chromatography, whilst KR100848132B1 describes a method for the simultaneous analysis of multicomponent derivatives of amphetamine and cannabis using gas chromatography.

The invention disclosed in patent specification CN112763289A relates to a kit and method for detecting amphetamine in hair using antibodies, based on a colour change.

Patent applications US5248791A and US5262333A describe an immunoenzymatic procedure utilising fluorescence polarisation for the simultaneous determination of amphetamine and d-methamphetamine.

The patent specification CN1 08181385A, however, describes a method for the simultaneous detection of R(-)-methamphetamine, S(+)-methamphetamine, R(-)-amphetamine and S(+)-amphetamine in liquid biological samples, involving sample pretreatment and analysis by LC-MS/MS.

Application No. CN100406882C relates to a capillary electrophoresis method with amperometric detection for amphetamines, whilst patent specification CN109655569A describes a method for detecting amphetamines in urine using solid-phase microextraction and gas chromatography.

The invention described in CN112213412A discloses a rapid screening method for multiple narcotic substances in hair, utilising solid-phase microextraction and liquid chromatography/mass spectrometry.

Based on an analysis of existing solutions gathered from patent databases, the inventors of this invention have established that no invention has yet been developed that describes the use of polarised liquid-liquid interfaces for the detection and quantification of amphetamine. No methods have been found that describe an electrochemical method for the determination of amphetamine.

The essence of the method for the determination of amphetamine in the presence of caffeine and paracetamol according to the invention consists in that, initially, an appropriately designed electrochemical cell, preferably made of glass or another material resistant to organic solvents, is provided; the cell comprises a lower part filled with an organic phase being a solution of a supporting electrolyte at least partially dissociating into ions with a low partition coefficient into the aqueous phase, and an upper part filled with an aqueous phase containing a solution of a supporting electrolyte being a salt dissociating into ions with a low partition coefficient into the organic phase; subsequently, two auxiliary electrodes, preferably made of platinum or stainless steel, and two reference electrodes in the form of a silver wire coated with Ag/AgCl are introduced. All the electrodes are then connected to a potentiostat, which enables potentiostatic or galvanostatic polarisation of the liquid-liquid interface. The sample of amphetamine under test is dissolved in an aqueous phase containing a buffer solution with a pH lower than the pKa of amphetamine. In the next step, an electrochemically controlled interfacial transfer of an ionised amphetamine molecule across the liquid-liquid phase boundary takes place, without the redox reactions characteristic of solid electrodes. The transfer of an ionised amphetamine molecule is recorded using electroanalytical methods such as voltammetry. The magnitude of the potential applied from an external polarisation source, which is necessary to transfer the amphetamine cation from one phase to the other, is a specific value that provides qualitative information. The quantitative data consists of recorded Faraday currents, the magnitude of which is directly proportional to the concentration of the ionised form of amphetamine in the test solution. The determination of the concentration of the amphetamine being tested is based on standard calibration methods.

According to the invention, the method for detecting the presence of amphetamine in a sample involves carrying out the analysis using cyclic voltammetry, comprising the following steps:
in the first step, equal volumes of 0.01 M acetic acid, 0.01 M boric acid and 0.01 M phosphoric acid are mixed, and the pH is adjusted to between 5 and 7 to prepare a Britton-Robinson buffer solution, which serves as the primary electrolyte;
in the second step, the organic phase is prepared by dissolving bis(triphenylphosphoranylidene)ammonium tetrakis-(4-chlorophenyl)borate in 1,2-dichloroethane or bis(triphenylphosphoranylidene)ammonium tetrakis-[3,5-bis(trifluoromethyl)phenyl]borate in nitrobenzene or tetradodecylammonium tetrakis(4-chlorophenyl)borate in 2-nitrophenyl n-octyl ether, such that the final concentration of the base electrolyte in the organic phase is ≥5 mM;
in the third step, the aqueous phase is prepared, consisting of a 25 µM solution of an internal calibration standard, preferably tetrapropylamine chloride, in a buffer at pH 5;
in the fourth step, the electrochemical cell is filled with an aqueous phase and an organic phase, whereby the two phases do not mix and a phase boundary forms between them, and then a silver chloride electrode, acting as the reference electrode, and a platinum electrode, acting as the auxiliary electrode, are introduced sequentially into the organic phase, whilst a silver chloride electrode, acting as the reference electrode, and a platinum electrode, acting as the auxiliary electrode, are introduced sequentially into the aqueous phase;
in the fifth step, the electrodes are connected to a potentiostat, and the reference electrodes are used to measure the potential drop at the liquid-liquid phase boundary, while the counter electrodes are used to measure the current flowing in the circuit, the moment of appearance of a current signal associated with a change in the value of the potential difference applied to the liquid-liquid interface from an external polarization source in each phase is observed, which indicates the presence of the calibration standard ion in the aqueous phase;
in the sixth step, a calibration curve is determined by repeating steps three to five, wherein the aqueous phase consists of an amphetamine solution in a buffer with a pH in the range of 2-6; with each successive repetition, the concentration of amphetamine in the aqueous phase is increased by 10 µM, and for each amphetamine concentration, the current-potential relationship is recorded;
in the seventh step, the aqueous phase is prepared by dissolving the sample under investigation in a buffer with a pH in the range of 2-6, steps three to five are then repeated, and the moment of change in the potential applied from an external polarization source in each phase indicates the presence of amphetamine in the tested sample;
if amphetamine is detected in the test sample, an additional eighth step is carried out, in which the difference between the capacitive current reading and the peak current value is correlated with the values determined from the calibration curve, and the concentration of amphetamine in the test sample is determined.

Preferably, in the method according to the invention, the calibration standard is tetrapropylammonium chloride in a buffer at pH 5, wherein the phase transfer potential for the tetrapropylammonium ion is -0.091 V.

Preferably, in the method according to the invention, the sample consists of a mixture of amphetamine with paracetamol and/or caffeine.

In another embodiment of the invention, the aqueous phase contains paracetamol at a concentration several thousand times higher than that of amphetamine.

In another embodiment of the invention, the aqueous phase contains caffeine at a concentration several thousand times higher than that of amphetamine

In another embodiment of the invention, the aqueous phase contains paracetamol and caffeine at a concentration several thousand times higher than that of amphetamine.

In another embodiment of the invention, the pH of the aqueous phase ranges from 1.0 to 8.0 in the presence of paracetamol in the aqueous phase.

In another embodiment of the invention, the pH of the aqueous phase ranges from 1.0 to 8.0 in the presence of caffeine in the aqueous phase.

In another embodiment of the invention, the pH of the aqueous phase ranges from 1.0 to 8.0 in the presence of paracetamol and caffeine in the aqueous phase.

The two-phase system used meets specific physicochemical requirements: the solvents of the two phases are mutually insoluble and have a dielectric constant that allows for the partial dissociation of basic electrolyte salts into ions, with the ions in the aqueous phase being highly hydrophilic and those in the organic phase being highly hydrophobic.

The main advantage of the method according to the invention is that amphetamine can be detected in street drug samples containing paracetamol and/or caffeine after the sample has been dissolved in an aqueous phase.

Furthermore, analysis of the sample is straightforward, taking no more than a few dozen seconds once the analyte has been introduced into the measurement system; it allows for the detection of amphetamine at concentrations as low as a few micromoles per litre; the analysis can be integrated with mobile devices; and the analysis is selective (the method for determining amphetamine has been verified in the presence of caffeine and paracetamol, and has been used to determine amphetamine in samples of drugs confiscated by the relevant authorities).

The invention is illustrated in the embodiments and in the drawing, where
[Fig. 1] shows a schematic diagram of an electrochemical cell;

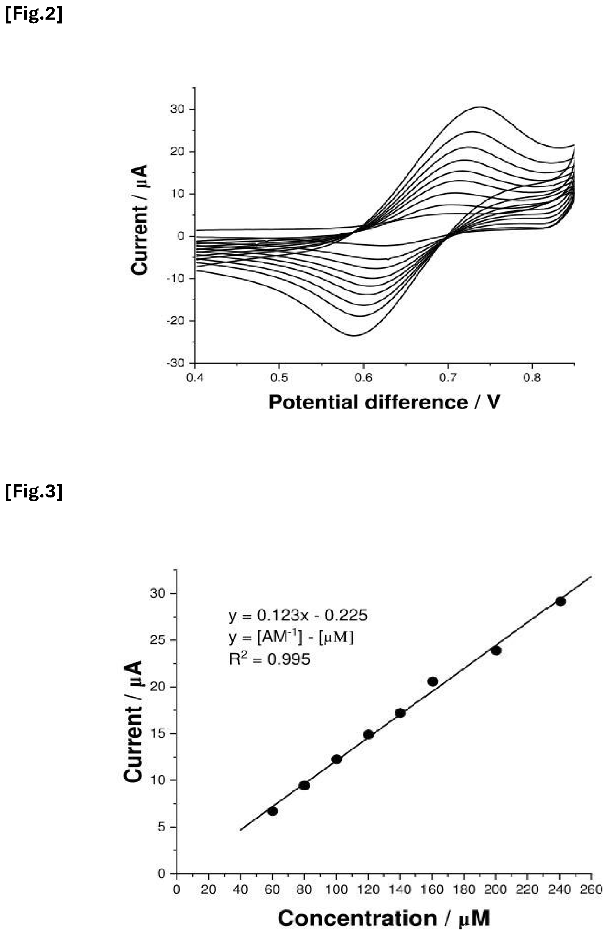

[Fig. 2] shows a current-potential curve recorded for increasing concentrations of amphetamine in the aqueous phase, providing electroanalytical information on the system under investigation;
[Fig. 3] presents a calibration curve used for the quantitative analysis of the amphetamine sample, derived from an analysis of the intensity of the current signals;
[Fig. 4] presents current-potential curves recorded for tetrapropylammonium ions at a concentration of 25 µM and amphetamine at a concentration of 25 µM at pH values of 2, 5, 7 and 11;
[Fig. 5] presents the current-voltage relationships recorded in the presence of amphetamine at a concentration of 25 µM, paracetamol at a concentration of 500 µM, and caffeine at concentrations ranging from 25 to 1000 µM;
[Fig. 6] presents the dependence of the positive and negative current associated with the interfacial transfer of amphetamine on the concentration of caffeine in the aqueous phase;
[Fig. 7] shows the current-voltage curve recorded for a genuine sample of amphetamine.

### Example 1

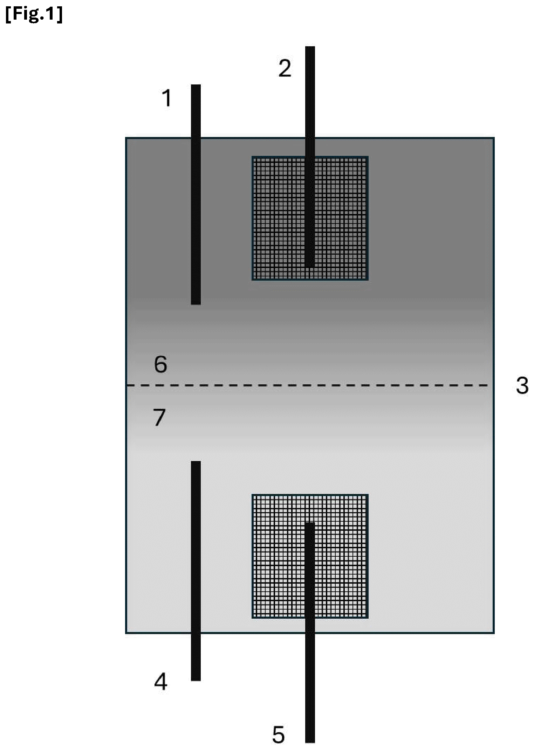

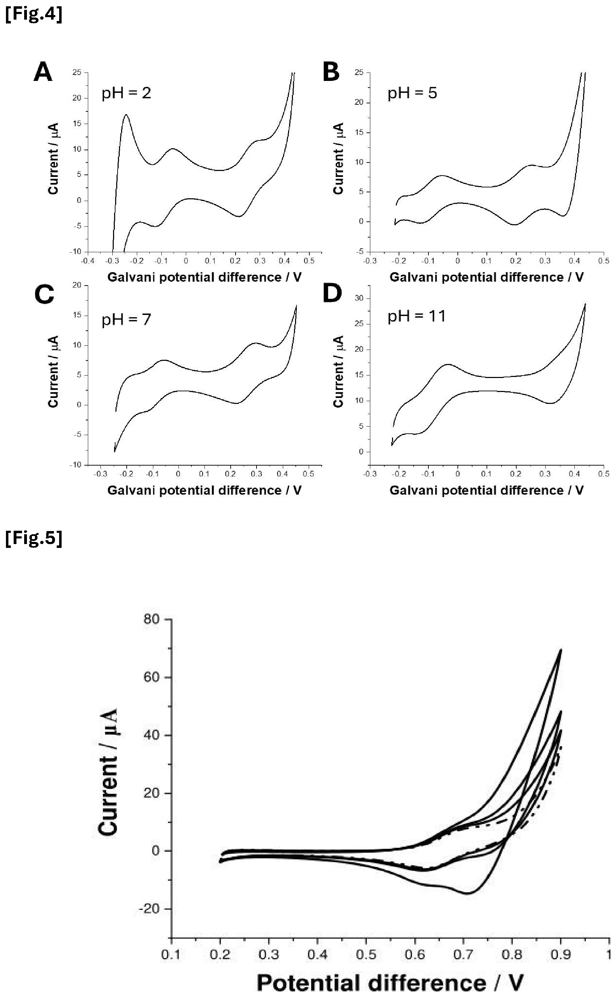

To a properly constructed electrochemical cell shown in [Fig. 1] consisting of a lower compartment 7 filled with an organic phase containing a solution of bis(triphenylphosphoranylidene)ammonium tetrakis-(4-chlorophenyl)borate dissolved in 1,2-dichloroethane, and an upper compartment 6 filled with an aqueous phase at pH 5 containing a solution of Britton-Robinson buffer, two pairs of electrodes were introduced. For both phases, two reference electrodes (1 and 4) in the form of an Ag wire coated with a layer of AgCl were used, along with two auxiliary electrodes (2 and 5) made of platinum. Once the electrodes had been correctly connected to the potentiostat, the liquid-liquid phase boundary 3 formed at the interface between the two phases became polarised. The technique used to detect amphetamine was cyclic voltammetry. Cyclic voltammograms were recorded in the presence of increasing concentrations of amphetamine dissolved in the aqueous phase, which consisted of a 10 mM Britton-Robinson buffer solution prepared in 10 mM NaCl at a pH of 5. The analysis of Faraday currents involved measuring the peak current and subtracting the measured capacitive current from the resulting numerical value, and then correlating the resulting numerical values with the concentration of amphetamine in the aqueous phase of the measurement system, which enabled a calibration curve to be plotted. The calibration curve was used to quantify the amphetamine content in the samples analysed. In order to determine the formal galvanic potential, a suitable volume of tetrapropylammonium chloride solution was added to the measuring cell so that its concentration in the aqueous phase was 25 µM. The formal phase-transfer potential of the tetrapropylammonium ion between the aqueous phase and the organic phase (1,2-dichloroethane) is -0.091 V. The tetrapropylammonium ion was used as an internal calibration standard. The transfer of amphetamine cations from the aqueous phase to the organic phase occurs at a Galvani potential difference of approximately 0.27 V at pH 5, as shown in [Fig. 4] The difference between the transfer potentials of the tetrapropylammonium ion and the amphetamine cation provided qualitative information about the amphetamine sample under investigation.

### Example 2

Proceeding as in Example 1, with the difference that the organic phase consisted of a solution of bis(triphenylphosphoranylidene)ammonium tetrakis-[3,5-bis(trifluoromethyl)phenyl]borate dissolved in nitrobenzene.

### Example 3

Proceeding as in Example 1, with the difference that the organic phase used was a solution of tetradodecylammonium tetrakis(4-chlorophenyl)borate dissolved in 2-nitrophenyl n-octyl ether.

### Example 4

Proceeding as in Example 1, with the difference that a different buffer with a pH of 2 was used as the aqueous phase.

### Example 5

Proceeding as in Example 1, with the difference that a different buffer with a pH of 3 was used as the aqueous phase.

### Example 6

Proceeding as in Example 1, with the difference that a different buffer with a pH of 4 was used as the aqueous phase.

### Example 7

Proceeding as in Example 1, with the difference that a different buffer with a pH of 6 was used as the aqueous phase.

### Example 8

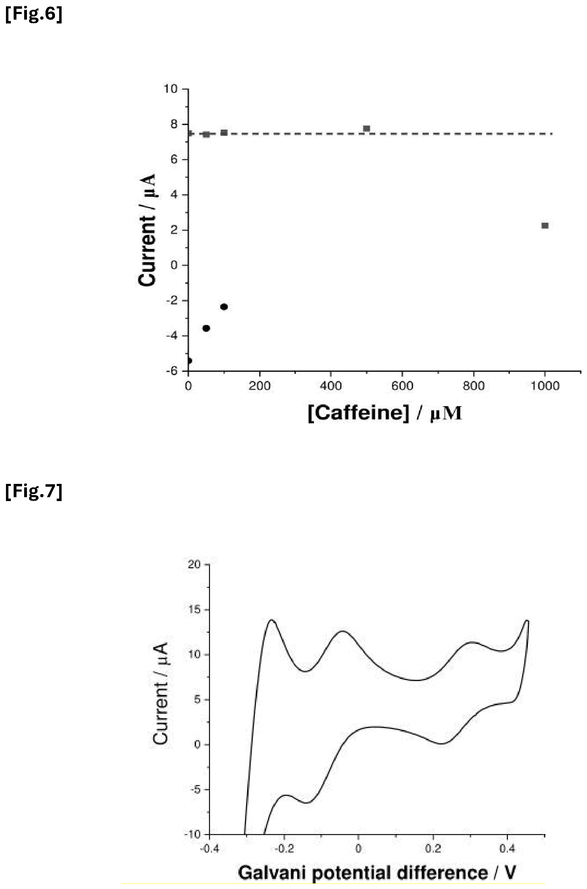

Proceeding as in Example 1, with the difference that paracetamol is present in the aqueous phase, as shown in [Fig. 5, 6].

### Example 9

Proceeding as in Example 1, with the difference that the aqueous phase contains caffeine at a concentration several hundred times higher than that of amphetamine, as shown in [Fig. 5, 6].

### Example 10

Proceeding as in Example 1, with the difference that the aqueous phase contains paracetamol and caffeine.

### Example 11

Proceeding as in Example 1, with the difference that a different internal standard, namely tetraethylamine, was used in the aqueous phase.

## Claims

1. The method for determining the presence of amphetamine in a sample, wherein the determination is carried out using cyclic voltammetry, comprising following steps:
- in the first step, equal volumes of 0.01 M acetic acid, 0.01 M boric acid and 0.01 M phosphoric acid are mixed, and the pH is adjusted to between 5 and 7 to prepare a Britton-Robinson buffer solution, which serves as the primary electrolyte;
- in the second step, the organic phase (6) is prepared by dissolving bis(triphenylphosphoranylidene)ammonium tetrakis-(4-chlorophenyl)borate in 1,2-dichloroethane or bis(triphenylphosphoranylidene)ammonium tetrakis-[3,5-bis(trifluoromethyl)phenyl]borate in nitrobenzene or tetradodecylammonium tetrakis(4-chlorophenyl)borate in 2-nitrophenyl n-octyl ether, such that the final concentration of the base electrolyte in the organic phase is ≥5 mM;
- in the third step, the aqueous phase (7) is prepared, consisting of a 25 µM solution of an internal calibration standard, preferably tetrapropylamine chloride, in a buffer at pH 5;
- in the fourth step, the electrochemical cell is filled with an aqueous phase (7) and an organic phase (6), whereby the two phases do not mix and a phase boundary (3) forms between them, and then a silver chloride electrode (1), acting as the reference electrode, and a platinum electrode (2), acting as the auxiliary electrode, are introduced sequentially into the organic phase (6), whilst a silver chloride electrode (4), acting as the reference electrode, and a platinum electrode (5), acting as the auxiliary electrode, are introduced sequentially into the aqueous phase (7);
- in the fifth step, the electrodes (1, 2, 4, 5) are connected to a potentiostat, and the electrodes (1, 4) are used to measure the potential drop at the liquid-liquid phase boundary, while the electrodes (2, 5) are used to measure the current flowing in the circuit, the moment of appearance of a current signal associated with a change in the value of the potential difference applied to the liquid-liquid interface from an external polarization source in each phase (6, 7) is observed, which indicates the presence of the calibration standard ion in the aqueous phase (7);
- in the sixth step, a calibration curve is determined by repeating steps three to five, wherein the aqueous phase (7) consists of an amphetamine solution in a buffer with a pH in the range of 2-6; with each successive repetition, the concentration of amphetamine in the aqueous phase is increased by 10 µM, and for each amphetamine concentration, the current-potential relationship is recorded;
- in the seventh step, the aqueous phase (7) is prepared by dissolving the sample under investigation in a buffer with a pH in the range of 2-6, steps three to five are then repeated, and the moment of change in the potential applied from an external polarization source in each phase (6, 7) indicates the presence of amphetamine in the tested sample;
- in case when amphetamine is detected in the test sample, an additional eighth step is carried out, in which the difference between the capacitive current reading and the peak current value is correlated with the values determined from the calibration curve, and the concentration of amphetamine in the test sample is determined.

2. The method according to claim 1, wherein the calibration standard is tetrapropylammonium chloride in a buffer at pH 5, and the interfacial transfer potential for the tetrapropylammonium ion is -0.091 V.

3. The method according to claim 1, wherein the sample consists of a mixture of amphetamine with paracetamol and/or caffeine.
